# EUROPEAN PATENT SPECIFICATION

(11) **EP 2 490 640 B1**
(45) Date of publication and mention of the grant of the patent: **18.06.2014**
(21) Application number: 10768489.6
(22) Date of filing: 21.10.2010
(51) Int. Cl.: A61F 15/00

(54) **AN APPLICATOR FOR APPLYING AN ARTICLE TO THE SKIN**
APPLIKATOR ZUM AUFTRAGEN EINES ARTIKELS AUF DIE HAUT
APPLICATEUR PERMETTANT D'APPLIQUER UN ARTICLE SUR LA PEAU

(30) Priority: 21.10.2009 DK 200901143
(43) Date of publication of application: 29.08.2012
(73) Proprietor: Ambu A/S, 2750 Ballerup (DK)
(72) Inventor: SONNENBORG, Frederik, DK-4040 Jyllinge (DK); BAY, Lasse, DK-2400 Copenhagen NV (DK)
(74) Representative: Nielsen, Kim Garsdal
(86) International application number: PCT/EP2010/065847
(87) International publication number: WO 2011/048171

(56) References cited:
- WO-A1-00/30579
- WO-A1-98/38955
- FR-A1- 2 848 535

## Description

The current invention relates to an applicator for applying an article to the skin of a patient.

More specifically the invention relates to such an applicator comprising a liner, an article carrying layer and an applicator layer, where the article carrying layer has a bottom and a top surface, both being delimited by the periphery of the carrying layer, wherein the bottom surface comprises an article carrying area and an adhesive area being releasably adhered to a surface on the liner by means of a first adhesive, wherein the applicator layer has a surface being releasably adhered to the top surface of the article and wherein the first and the second adhesives are selected so that, when the applicator layer is pulled away from the liner in an area where the applicator layer and the second adhesive covers the first part of the periphery of the article carrying layer, then the first adhesive releases the liner, and the article carrying layer sticks to the applicator layer.

### Description of related art

An example of an applicator similar to the one mentioned in the opening paragraph is disclosed in US 7,521,586.

This document discloses a package or applicator allowing a patient to apply an adhesive bandage carrying an article e.g. being a gauze pad being treated with a chemical compound such as nicotine for smokers or nitroglycerine for heart patients. The use of the package according to this document provides the possibility of applying the bandage by only using one hand.

### Summary of the invention

It is a first aspect of the current invention to provide an applicator, having improved properties to be used e.g. for neonatal patients and others, having very sensitive or uneven skin.

This aspect is provided by an applicator as mentioned in the opening paragraph of claim 1, further having the first part of the top surface on the article carrying layer, being adhered to the applicator layer, extends at least partly over or at least on two opposite sides of the article carrying area.

The present invention hereby ensures that the article placed underneath the article carrying layer is efficiently supported by the applicator layer until the article carrying layer is applied to the skin of a patient, where after the applicator layer can be removed from the article carrying layer.

Hereby it is possible to select materials for the article carrying layer, being more friendly to the sensitive skin of e.g. a neonatal patient, and e.g. having reduced stiffness or thickness, so that it is easier to apply the article correctly to the skin of the patient without the risk of creating unnecessary wrinkles or damage to the adhesive on the article carrying layer before it is applied to the skin.

In a preferred embodiment the article carried by the carrying layer is an electrode to be used for monitoring e.g. the specific physical conditions of a patent. In this relation the present invention is extremely important as it is necessary to keep a good contact between the electrode and the skin of the patient, so that the risk of faulty monitoring is minimized.

In this relation the term electrode covers all articles that are suitable for conducting electrical current from the surface of the skin of a patient and to another place, e.g. via an electrical conductor.

The above mentioned advantage is, however, further improved when the electrode comprises an electrically conducting gel, so that even the electrode is flexible to a certain extend.

The present invention is also extremely advantageous in situations where the article comprises a bandage only and especially in situations where the article comprises one or more chemical compounds for treating the patient. In these situations it is namely also important to ensure a stable and close contact between the article and the skin of the patient.

In a preferred embodiment the applicator layer comprises a first pulling tab extending from the applicator layer, where it is adhered to the first part of the periphery of the article carrying layer. In this way it is possible to apply the carrying layer to the skin of the patient by only using a single hand, and still with a minimized risk of having wrinkles or damages to the carrying layer and the adhesive applied to it.

In this relation it is further advantageous if the applicator layer comprises a second pulling tab extending from the applicator layer at a place where it is adhered to the first part of the top surface of the article carrying layer and/or from the applicator layer where it is adhered to another part of the periphery of the article carrying layer than where the first pulling tab extends.

For production purposes it is especially advantageous if the major part of the article carrying area is placed between the first and the second pulling tab. Hereby it is easily possible to arrange three or more carrying layers, side by side, on a single liner.

The risk of creating wrinkles and damages to the carrying layer when applying it to the skin of a patient, is further reduced if the first part of the top surface of the article carrying layer, is separated by a boundary from the remaining part of the top surface of the article carrying layer, and in that the second pulling tab at least partly extends from this boundary. This provides that pulling off the second pulling tab, results primarily in pulling forces in the area of the boundary, and not in the periphery of the carrying layer, and hence minimizes the risk that the carrying layer releases the skin of the patent.

In order to ensure correct use of the applicator, then it is especially advantageous if the applicator on e.g. the first and the second pulling tab, are marked with indicators, e.g. numbers, showing to the user that the tabs should be used by puling the first pulling tab before pulling the second pulling tab.

By having the adhesive on the surface of the applicator layer extending in the area of at least one pulling tab, and in the same time having a cover being adhered to this area, then it is ensured that the adhesive will only be sticking to the other parts of the applicator in areas where the cover is not present, and hence a precise function of the adhesive is obtained, so that it further ensures safe handling of the applicator, with the risk of creating wrinkles or damages to the carrying layer before applying it to the skin of the patient.

For the same reason it is further advantageous if the surface of the liner is made from a material providing easy releasing properties with respect to the first adhesive on the article carrying layer, and/or if the first and the second adhesives are selected so that when the article carrying layer is adhered to the skin of a patient, then the second adhesive releases the applicator layer from the article carrying layer, when pulling the applicator layer away from the article carrying layer.

The risk of creating wrinkles on the article carrying layer is further reduced if it has a central part comprising the article carrying area, and a number of wings extending from the central part and outwardly.

In this relation, and for the same purpose the first part of the top surface of the article carrying layer can be separated by a boundary from the remaining part of the top surface of the article carrying layer, and at least one of the wings can form a part of the remaining part of the top surface of the article carrying layer.

### Brief description of the drawings

In the following, the invention will be described in greater detail with reference to embodiments shown by the enclosed figures. It should be emphasized that the embodiments shown are used for example purposes only and should not be used to limit the scope of the invention.
Figure 1 shows a complete top view of a package comprising 3 applicators according to the present invention.
Figures 2 to 6 are all schematic side views of the same applicator according to the invention, but shown in different states of use.

### Detailed description of the embodiments:

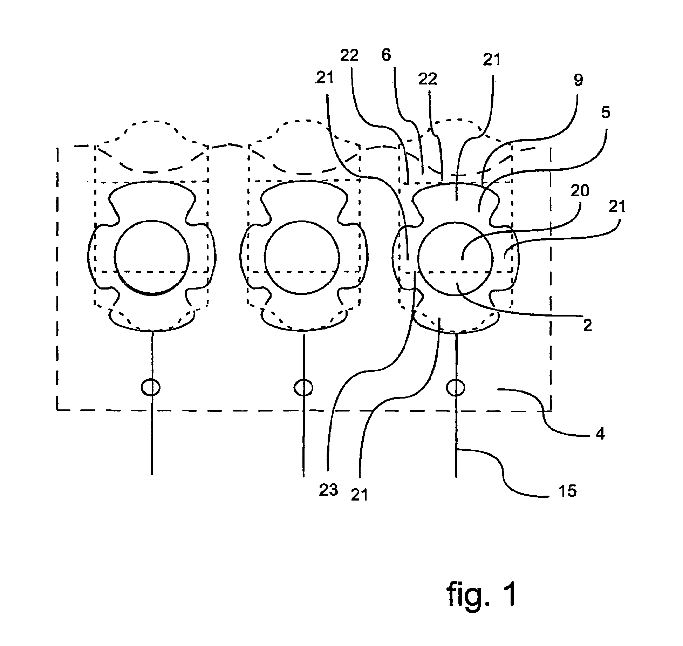

Figure 1 shows a package comprising 3 identical applicators 1 according to the invention. The package comprises a liner 4 in the form of a single sheet functioning as a liner 4 for all applicators. The liner 4 is illustrated with punctured lines, and 3 carrying layers 5, each illustrated with their periphery 9 shown in full line, are adhered upon the liner 4.

On top of each carrying layer 4 is adhered one applicator layer 6, illustrated with dotted lines. Each applicator layer 6 is adhered to the top surface 8 of the carrying layer 5 between the dotted lines 22 and 23, so that the remaining parts of the applicator layer 6 forms a first pulling tab 16 and a second pulling tab 17.

The article carrying layer 5 is formed by a central part 20 where the article 2 is fixed to the article carrying layer 5, and on four sides, there is a wing 21 that extends outwardly from the centre 20. Hereby the article carrying layer 5 is possible to fasten to very uneven surfaces, such as the skin of a newborn child, without the risk of creating wrinkles on the article carrying layer 5

In the following the function of the pulling tabs are described in more detail, but at figure 1 it can be seen that the first 16 and the second 17 pulling tab are numbered as number 1 and 2 respectively in order to indicate to the user, that at first the user shall pull the first pulling tab 16 away from the package in order to release the carrying layer 5 from the liner 4 first, and hereafter the second pulling tab 17 can be pulled away from off the carrying layer 5, after the carrying layer has been adhered to e.g. the skin of a patient, and so that the applicator layer 6 is pulled off the carrying layer 5.

In the embodiment shown in fig. 1 the carrying layer 5 carries an article 2 in the form of an electrode for monitoring e.g. a specific physical condition of a patient. It is, however, possible to use exactly the same design and construction of the applicator 1, but where the article 2, instead of an electrode, comprises.e.g.. a gauze sheet or a chemical compound for treating a patient.

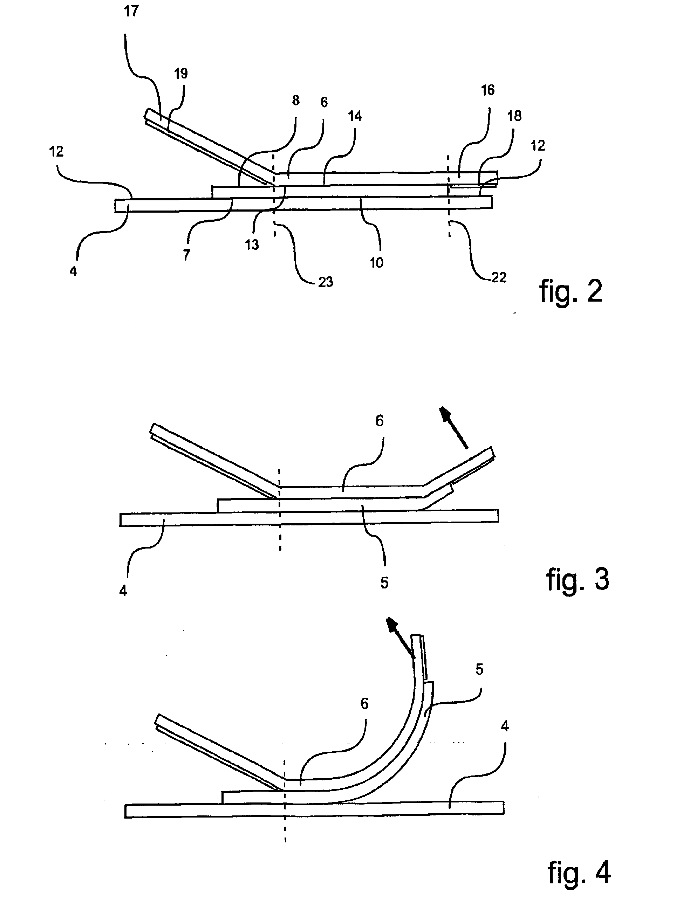

In the following figures 2 to 6 a schematic side view of one applicator according to the invention is shown.

In fig. 2 the applicator is shown in a ready to use state, where the liner 4 is shown as the lowermost sheet, having a surface 12 facing the article carrying layer 5. The liner is made of, or covered by, a material that releases the first adhesive (not shown) arranged between the bottom surface 7 of the carrying layer 5 and the surface 10 of the liner, and on the top surface 8 of the carrying layer the applicator layer 6 is adhered, by means of a second adhesive (not shown) to the carrying layer 5 between the dotted lines 22 and 23.

For the purpose of easy understanding of the principle of the present invention the article 2 is not shown in figure 2 to 6, but in accordance with the invention a major part of the article 2 is fixed to the bottom surface 10 of the article carrying layer 5, so that the major part of the article is situated between the dotted lines 22 and 23.

For production reasons, and in order to ensure that the adhesive is effective, then the second adhesive is distributed over the complete surface 13 of the applicator layer. Therefore, in order to ensure that the first 16 and the second 17 pulling tab does not adhere to anything else, the covers 18, 19 are adhered instead for covering these areas.

In figure 3 the applicator is hereafter seen in a situation where the first pulling tab 16 is lifted away, as illustrated by the arrow, from the liner 4, and hence, as the surface 12 of the liner 4 releases the first adhesive on the article carrying layer 5, the article carrying layer 5 is lifted off the liner 4.

Further pulling the first pulling tab 16 away from the liner 4, results in the situation as shown in fig. 4, where it can be seen that almost all the article carrying layer 5, is released from the liner 4.

As mentioned above, the article 2 (not shown) is located primarily between the dotted lines in fig. 2. This means that the same article 2 in the situation as shown in fig. 4, is carried by the article carrying layer 5, but also by the applicator layer 6, so that it is not only the article carrying layer 5 that carries the article 2. This provides a great degree of freedom for selecting materials for the article carrying layer, because the strength of this layer does not necessarily have to be sufficient to carry the article 2 alone. At the same time, the applicator layer helps keeping the article carrying layer flat, thereby minimizing the risk of creating wrinkles or damage to the article carrying layer and the first adhesive.

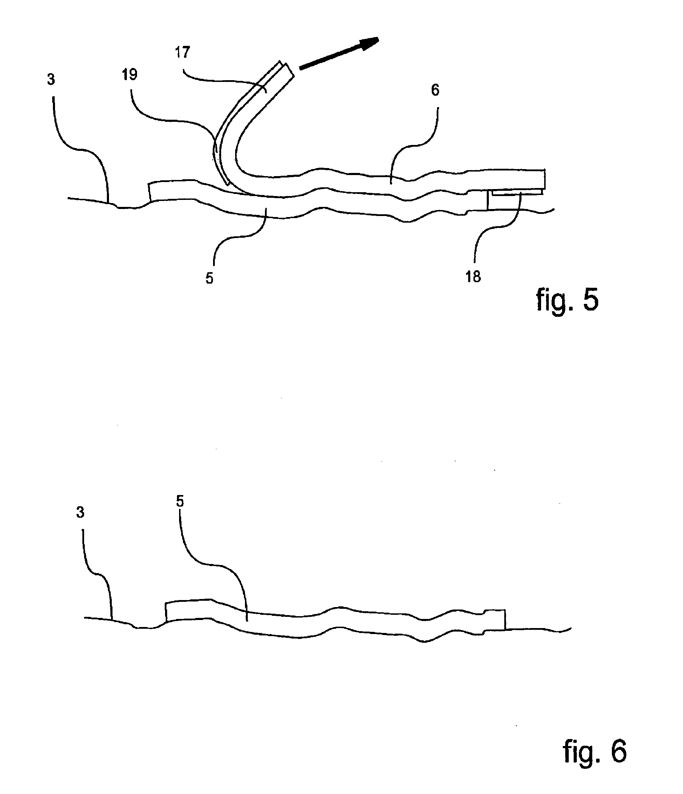

After having completely released the article carrying layer 5 from the liner 4, then the article carrying layer can easily be manipulated by the user by only using one hand, and the article carrying layer can be transferred e.g. to the skin 3 of a patent as shown in fig. 5, where the user now pulls the second pulling tab 17 away from the article carrying layer, and the article carrying layer sticks to the skin as a consequence of the selection of the adhesive, and the fact that a large part of the article carrying layer 5, due to the basic principle of the invention is situated on both sides of the area where the pulling forces applied to the article carrying layer 5 is concentrated.

The final result of the application process is shown in figure 6, where the applicator layer 6 is completely pulled away from the article carrying layer 5, so that the only parts left on the skin 3 of the patient, is the article carrying layer 5, and the article 2 (not shown).

It will be evident to the person skilled in the art that other embodiments are derivable from the teachings of this specification and that these embodiments should.also be included within the scope of protection. For example it is possible to alter the shape of the periphery of the article carrying layer, so that it may e.g. be round, elliptical or rectangular. Furthermore it is evident to the skilled person that there are many different possible combinations of adhesives and materials for the liner 4 and the layers 5, 6 that would fulfil the functions as mentioned above.

## Claims

1. An applicator (1) for applying an electrode (2) to the skin (3) of a patient comprising
- a liner (4);
- an article carrying layer (5); and
- an applicator layer (6),
where the article carrying layer (5) has a bottom (7) and a top (8) surface, both being delimited by a periphery (9) of the carrying layer (5), wherein the bottom surface (7) comprises
- an article carrying area (10) where the electrode (2) is fixed to the carrying layer (5), and
- an adhesive area (11) being releasably adhered to a surface (12) on the liner (4) by means of a first adhesive,
wherein the applicator layer (6) has a surface (13) being releasably adhered to only a first part (14) of the top surface (8) of the article carrying layer (5) and its periphery (9) by means of a second adhesive, and
wherein the first and the second adhesives are selected so that, when the applicator layer (6) is pulled away from the liner (4) in an area where the applicator layer (6) and the second adhesive covers the first part of the periphery of the article carrying layer (5), then the first adhesive releases the liner (4), and the article carrying layer (5) sticks to the applicator layer (6),
**characterized in that** the first part (14) of the top surface (8) on the article carrying layer (5), being adhered to the applicator layer (6), extends at least partly over or at least on two opposite sides of the article carrying area (10).

2. An applicator according to claim 1, **characterized in that** the electrode comprises an electrically conducting gel.

3. An applicator according to claim 1, **characterized in that** the applicator layer (6) comprises a first pulling tab (16) extending from the applicator layer (6), where it is adhered to the first part of the periphery of the article carrying layer (5).

4. An applicator according to claim 3, **characterized in that** the applicator layer (6) comprises a second pulling tab (17) extending from the applicator layer (6) at a place where it is adhered to the first part of the top surface of the article carrying layer and/or from the applicator layer (6) where it is adhered to another part of the periphery of the article carrying layer (5) than where the first pulling tab (16) extends.

5. An applicator according to claim 4, **characterized in that** the major part of the article carrying area (10) is placed between the first (16) and the second pulling tab (17).

6. An applicator according to claim 4 or 5, **characterized in that** the first part of the top surface of the article carrying layer (5), is separated by a boundary from the remaining part of the top surface (8) of the article carrying layer (5), and **in that** the second pulling tab (17) at least partly extends from this boundary.

7. An applicator according to claim 4, 5 or 6, **characterized in that** the first and the second pulling tab (16, 17) are marked with indicators showing to the user that the tabs should be used by puling the first pulling tab (16) before pulling the second pulling tab (17).

8. An applicator according to claim 3 or 4, **characterized in that** the adhesive on the surface of the applicator layer (6) extends in the area of at least one pulling tab (16, 17), and **in that** a cover (18, 19) is adhered to this area, so that the adhesive on the pulling (16, 17) tab does not adhere to the article carrying layer (5) or the liner (4).

9. An applicator according to one or more of the preceding claims, **characterized in that** the first and the second adhesives are selected so that when the article carrying layer (5) is adhered to the skin (3) of a patient, then the second adhesive releases the applicator layer (6) from the article carrying layer (5), when pulling the applicator layer (6) away from the article carrying layer (5).

10. An applicator according to one or more of the preceding claims, **characterized in that** the article carrying layer (5) has a central part (20) comprising the article carrying area (10), and where a number of wings (21) extends from the central part (20) and outwardly.

11. An applicator according to claim 10 **characterized in that** the first part (14) of the top surface (8) of the article carrying layer (5), is separated by a boundary from the remaining part of the top surface (8) of the article carrying layer (5), and where at least one of the wings (21) forms part of the remaining part of the top surface (8) of the article carrying layer (5).

## Patentansprüche

1. Applikator (1) zum Auftragen einer Elektrode (2) auf die Haut (3) eines Patienten, wobei der Applikator Folgendes umfasst:
- eine Decklage (4);
- eine Artikelträgerschicht (5); und
- eine Applikatorschicht (6),
wobei die Artikelträgerschicht (5) eine untere (7) und eine obere (8) Oberfläche hat, die beide durch einen Umkreis (9) der Trägerschicht (5) begrenzt sind,
wobei die untere Oberfläche (7) Folgendes umfasst:
- eine Artikelträgerfläche (10), wo die Elektrode (2) an der Trägerschicht (5) fixiert ist; und
- eine Klebefläche (11), die mittels eines ersten Klebers an einer Oberfläche (12) auf der Decklage (4) lösbar anhaftet,
wobei die Applikatorschicht (6) eine Oberfläche (13) hat, die mittels eines zweiten Klebers nur an einem ersten Teil (14) der oberen Oberfläche (8) der Artikelträgerschicht (5) und an ihrem Umkreis (9) anhaftet, und
wobei der erste und der zweite Kleber so ausgewählt sind, dass, wenn die Applikatorschicht (6) von der Decklage (4) in einem Bereich abgezogen wird, in dem die Applikatorschicht (6) und der zweite Kleber den ersten Teil des Umkreises der Artikelträgerschicht (5) abdecken, der erste Kleber die Decklage (4) freigibt und die Artikelträgerschicht (5) an der Applikatorschicht (6) anhaftet,
**dadurch gekennzeichnet, dass** der erste Teil (14) der oberen Oberfläche (8) an der Artikelträgerschicht (5), die an der Applikatorschicht (6) anhaftet, sich mindestens teilweise über oder mindestens an zwei gegenüberliegende(n) Seiten der Artikelträgerfläche (10) erstreckt.

2. Applikator nach Anspruch 1, **dadurch gekennzeichnet, dass** die Elektrode ein elektrisch leitendes Gel umfasst.

3. Applikator nach Anspruch 1, **dadurch gekennzeichnet, dass** die Applikatorschicht (6) eine erste Zuglasche (16) umfasst, die sich von der Applikatorschicht (6) aus an einer Stelle erstreckt, wo sie am ersten Teil des Umkreises der Artikelträgerschicht (5) anhaftet.

4. Applikator nach Anspruch 3, **dadurch gekennzeichnet, dass** die Applikatorschicht (6) eine zweite Zuglasche (17) umfasst, die sich von der Applikatorschicht (6) aus an einer Stelle erstreckt, wo sie am ersten Teil der oberen Oberfläche der Artikelträgerschicht anhaftet und/oder sich von der Applikatorschicht (6) aus an einer Stelle erstreckt, wo sie, im Gegensatz zur ersten Zuglasche (16), an einem anderen Teil des Umkreises der Artikelträgerschicht (5) anhaftet.

5. Applikator nach Anspruch 4, **dadurch gekennzeichnet, dass** der Hauptteil der Artikelträgerfläche (10) zwischen der ersten (16) und der zweiten (17) Zuglasche platziert ist.

6. Applikator nach Anspruch 4 oder 5, **dadurch gekennzeichnet, dass** der erste Teil der oberen Oberfläche der Artikelträgerschicht (5) durch eine Abgrenzung vom verbleibenden Teil der oberen Oberfläche (8) der Artikelträgerschicht (5) getrennt ist und dass die zweite Zuglasche (17) sich mindestens teilweise von dieser Abgrenzung aus erstreckt.

7. Applikator nach Anspruch 4, 5 oder 6, **dadurch gekennzeichnet, dass** die erste und die zweite Zuglasche (16, 17) mit Kennzeichen markiert sind, die dem Benutzer zeigen, dass zuerst die erste Zuglasche (16) und erst danach die zweite Zuglasche (17) abgezogen werden soll.

8. Applikator nach Anspruch 3 oder 4, **dadurch gekennzeichnet, dass** sich der Kleber an der Oberfläche der Applikatorschicht (6) in den Bereich von mindestens einer Zuglasche (16, 17) erstreckt und dass eine Abdeckung (18, 19) an diesem Bereich anhaftet, so dass der Kleber an der Zuglasche (16, 17) nicht an der Artikelträgerschicht (5) oder an der Decklage (4) anhaftet.

9. Applikator nach einem oder mehreren der vorstehend aufgeführten Ansprüche, **dadurch gekennzeichnet, dass** der erste und der zweite Kleber so ausgewählt sind, dass, wenn die Artikelträgerschicht (5) an der Haut (3) eines Patienten anhaftet, der zweite Kleber die Applikatorschicht (6) von der Artikelträgerschicht (5) freigibt, wenn die Applikatorschicht (6) von der Artikelträgerschicht (5) abgezogen wird.

10. Applikator nach einem oder mehreren der vorstehend aufgeführten Ansprüche, **dadurch gekennzeichnet, dass** die Artikelträgerschicht (5) einen zentralen Teil (20) hat, der die Artikelträgerfläche (10) umfasst, wobei sich eine Anzahl von Flügeln (21) vom zentralen Teil (20) aus und nach außen erstrecken.

11. Applikator nach Anspruch 10, **dadurch gekennzeichnet, dass** der erste Teil (14) der oberen Oberfläche (8) der Artikelträgerschicht (5) durch eine Abgrenzung vom verbleibenden Teil der oberen Oberfläche (8) der Artikelträgerschicht (5) getrennt ist, wobei mindestens einer der Flügel (21) einen Teil des verbleibenden Teils der oberen Oberfläche (8) der Artikelträgerschicht (5) bildet.

## Revendications

1. Applicateur (1) permettant d'appliquer une électrode (2) sur la peau (3) d'un patient, comprenant
- une doublure (4) ;
- une couche de support d'article (5) ; et
- une couche d'applicateur (6),
la couche de support d'article (5) présentant une surface inférieure (7) et une surface supérieure (8), toutes deux étant délimitées par une périphérie (9) de la couche de support (5), la surface inférieure (7) comprenant
- une zone de support d'article (10), l'électrode (2) étant fixée à la couche de support (5), et
- une zone adhésive (11) collée de manière détachable à une surface (12) sur la doublure (4) au moyen d'un premier adhésif,
la couche d'applicateur (6) ayant une surface (13) collée de manière détachable sur seulement une première partie (14) de la surface supérieure (8) de la couche de support d'article (5) et sa périphérie (9) au moyen d'un deuxième adhésif, et les premiers et deuxièmes adhésifs étant choisis de telle sorte que lorsque la couche d'applicateur (6) est retirée de la doublure (4) dans une zone dans laquelle la couche d'applicateur (6) et le deuxième adhésif couvrent la première partie de la périphérie de la couche de support d'article (5), le premier adhésif libère la doublure (4), et la couche de support d'article (5) colle à la couche d'applicateur (6),
**caractérisé en ce que** la première partie (14) de la surface supérieure (8) sur la couche de support d'article (5), qui est collée à la couche d'applicateur (6), s'étend au moins en partie sur ou au moins sur deux côtés opposés de la couche de support d'article (10).

2. Applicateur selon la revendication 1, **caractérisé en ce que** l'électrode comprend un gel électriquement conducteur.

3. Applicateur selon la revendication 1, **caractérisé en ce que** la couche d'applicateur (6) comprend une première languette de traction (16) s'étendant depuis la couche d'applicateur (6), où elle est collée à la première partie de la périphérie de la couche de support d'article (5).

4. Applicateur selon la revendication 3, **caractérisé en ce que** la couche d'applicateur (6) comprend une deuxième languette de traction (17) s'étendant depuis la couche d'applicateur (6) à un endroit où elle est collée à la première partie de la surface supérieure de la couche de support d'article et/ou depuis la couche d'applicateur (6) où elle est collée à une autre partie de la périphérie de la couche de support d'article (5) que là où s'étend la première languette de traction (16).

5. Applicateur selon la revendication 4, **caractérisé en ce que** la majeure partie de la zone de support d'article (10) est placée entre la première (16) et la deuxième (17) languette de traction.

6. Applicateur selon la revendication 4 ou 5, **caractérisé en ce que** la première partie de la surface supérieure de la couche de support d'article (5) est séparée par une limite de la partie restante de la surface supérieure (8) de la couche de support d'article (5), et **en ce que** la deuxième langue de traction (17) s'étend au moins en partie depuis cette limite.

7. Applicateur selon la revendication 4, 5 ou 6, **caractérisé en ce que** la première et la deuxième langue de traction (16, 17) sont munies de marquages indicateurs montrant à l'utilisateur que les languettes devraient être utilisées en tirant sur la première languette de traction (16) avant de tirer sur la deuxième languette de traction (17).

8. Applicateur selon la revendication 3 ou 4, **caractérisé en ce que** l'adhésif sur la surface de la couche d'applicateur (6) s'étend dans la région d'au moins une languette de traction (16, 17), et **en ce qu'**un recouvrement (18, 19) est collé à cette région, de telle sorte que l'adhésif sur la languette de traction (16, 17) ne colle pas à la couche de support d'article (5) ou à la doublure (4).

9. Applicateur selon l'une ou plusieurs des revendications précédentes, **caractérisé en ce que** les premier et deuxième adhésifs sont choisis de telle sorte que lorsque la couche de support d'article (5) est collée à la peau (3) d'un patient, le deuxième adhésif libère la couche d'applicateur (6) de la couche de support d'article (5), lorsque l'on tire sur la couche d'applicateur (6) à l'écart de la couche de support d'article (5).

10. Applicateur selon l'une ou plusieurs des revendications précédentes, **caractérisé en ce que** la couche de support d'article (5) présente une partie centrale (20) comprenant la zone de support d'article (10), un certain nombre d'ailes (21) s'étendant depuis la partie centrale (20) et vers l'extérieur.

11. Applicateur selon la revendication 10, **caractérisé en ce que** la première partie (14) de la surface supérieure (8) de la couche de support d'article (5) est séparée par une limite de la partie restante de la surface supérieure (8) de la couche de support d'article (5), au moins l'une des ailes (21) faisant partie de la partie restante de la surface supérieure (8) de la couche de support d'article (5).
